# EUROPEAN PATENT APPLICATION

(11) **EP 4 303 558 A1**
(43) Date of publication of application: **10.01.2024**
(21) Application number: 23183918.4
(22) Date of filing: 06.07.2023
(51) Int. Cl.: G01N 1/22

(54) **AEROSOL DETECTION SYSTEM AND METHOD**

(30) Priority: 07.07.2022 US 202263367893 P; 29.08.2022 US 202263402030 P; 23.09.2022 US 202217952131
(71) Applicant: Hamilton Sundstrand Corporation, Charlotte, NC 28217-4578 (US)
(72) Inventor: GARDNER, Benjamin D., Colton, CA, 92324 (US)
(74) Representative: Dehns

(57) **Abstract**

In accordance with at least one aspect of this disclosure, a system includes one or more collection units (210) configured to collect a sample from an aerosol cloud and a detection unit (110) configured to analyze a composition the sample from the aerosol cloud. In certain embodiments, the one or more collection units can include a plurality of collection units. In embodiments, the collection unit can include a collection medium (214) configured to collect aerosol particles from the aerosol cloud and condense or trap the aerosol particles on or in the collection medium.

## Description

### TECHNICAL FIELD

The present disclosure relates to aerosol detection systems, and more particularly to chemical and/or aerosol detection systems.

### BACKGROUND

There is a need within the national security environment to detect aerosolized chemical threats over a battlespace, or even urban area. The need includes rapid identification of chemical cloud constituents as well as mapping of cloud dimensions and direction of travel. As a practical matter there is also a strong desire to have the detection system be a "standoff" type wherein the detection system does not come in contact with the cloud and thus become contaminated. Keeping the system free of contamination from chemical weapons agents (CWAs) or other dangerous chemicals eliminates potential post-mission hazards to personnel as they recover the system for reuse.

To date, while several chemical detection systems have been flown on UAVs and have demonstrated the detection of aerosols, there are no suitable systems that can provide both true standoff capability and strong chemical identification capability. The most capable systems (e.g., mobility spectrometry and mass spectrometry) work but require contact with the sample and result in a contaminated system. Optically based systems can be operated from a safe distance but do not have strong detection limits and have limited detection specificity, and to date, these systems only work analyzing liquid or solid chemicals on stationary surfaces. Chemicals found in aerosol clouds can be highly dispersed and the light detected from them is also highly dispersed, so the limits of detection are greatly diminished.

There remains a need in the art for improvements to chemical aerosol detection in the aerospace industry. This disclosure provides a solution for this need.

### SUMMARY

In accordance with at least one aspect of this disclosure, a system includes one or more collection units configured to collect a sample from an aerosol cloud and a detection unit configured to analyze a composition the sample from the aerosol cloud. In embodiments, a base unit configured to dispatch the one or more collection units can be included. In certain embodiments, the one or more collection units can include a plurality of collection units.

In embodiments, the collection unit can include a collection medium configured to collect aerosol particles from the aerosol cloud and condense or trap the aerosol particles on or in the collection medium. In certain embodiments, the collection module can further include a pump configured to draw aerosol particles from the aerosol cloud onto the collection medium. In certain embodiments, the collection medium can include a filter medium.

In certain embodiments, the collection unit can also include a sensor configured to detect hazardous particles in the collection medium and to provide a warning to the detection unit that a respective collection unit has entered a hazardous aerosol cloud. In embodiments, the sensor can include an optical sensor or spectrometer.

In embodiments, the detection unit can include an optical detection module having a radiation source configured to transmit an interrogation beam to remotely interrogate a respective collection medium of a respective collection unit, and a radiation receiver configured to receive a return signal from the respective collection medium. In embodiments, the optical detection module can also include a composition determination module configured to receive data from the radiation receiver to determine a chemical composition of the particles in the collection medium.

In embodiments, a standoff distance between the detection unit and the respective collection unit is sufficient such that exposure of the detection unit to the aerosol cloud is minimized. In certain embodiments, one or more of the detection unit and/or the one or more collection units can be configured to couple to a moving platform so as to define a mobile detection system. In such embodiments, the detection unit and the one or more collection units can each further include a respective communications module configured to communicate with a controller to guide the detection unit and the one or more collection units to one or more respective positions in space relative to the aerosol cloud. In certain embodiments, the controller can be or include a remote and/or on-ground controller. In embodiments, the respective communications modules can be configured to communicate a respective position of the detection unit to the one or more collections units to maintain the standoff distance. In embodiments, the respective communications module of the detection unit can also be configured to communicate the composition of the aerosol cloud to the controller and/or a user.

In embodiments, the detection unit and the one or more collection units can each further include a respective navigation module configured to guide the detection unit and the one or more collection units to one or more positions in space relative to the aerosol cloud without communication from a remote or on-ground controller. In certain embodiments, the detection unit and/or the one or more collection unit can include at least some autonomous control with respect to positioning relative to the aerosol cloud and in relation to nearby mobile platforms included in the system.

In accordance with at least one aspect of this disclosure, a method, includes dispatching a plurality of collection units towards an aerosol cloud, detecting, with a sensor disposed within a respective collection unit, an edge of the aerosol cloud, and collecting sample from the aerosol cloud in a collection medium within a respective collection unit. The method can further include condensing or trapping the aerosol particles within the sample on or in the collection medium and optically interrogating, with a detection unit, the aerosol particles on or in the collection medium. The method further includes, determining a chemical composition of the aerosol particles in the collection medium and communicating the chemical composition of the aerosol particles to a user.

In embodiments, remotely collecting can further include drawing a portion of the aerosol cloud onto a collection unit, wherein the collection unit includes the collection medium disposed therein.

In embodiments, optically interrogating can further include positioning the detection unit having an optical detection module operatively connected thereto proximate the aerosol cloud separated by a standoff distance and positioning a respective collection unit having collected the aerosol particles at a location between the aerosol cloud and the detection unit.

In embodiments, the method can further include moving the respective collection unit away from the aerosol cloud while the optical detection module determines the chemical composition, re-positioning the collection unit in the aerosol cloud and repeating the method or homing the collection unit to a base, and initiating a decontamination and/or destruction protocol based at least in part of the chemical composition of the aerosol particles.

These and other features of the embodiments of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below with reference to certain figures, wherein:
Fig. 1 is schematic view of an embodiment of aerosol collection and detection system in accordance with this disclosure;
Fig. 2 is an embodiment of another aerosol collection device for the system of Fig. 1, in accordance with this disclosure
Fig. 3 is a schematic diagram of an embodiment of a stationary aerosol collection and detection system in accordance with this disclosure; and
Fig. 4 is a schematic environmental depiction of the system of Fig. 3, showing the stationary aerosol collection and detection system in an urban environment.

### DETAILED DESCRIPTION

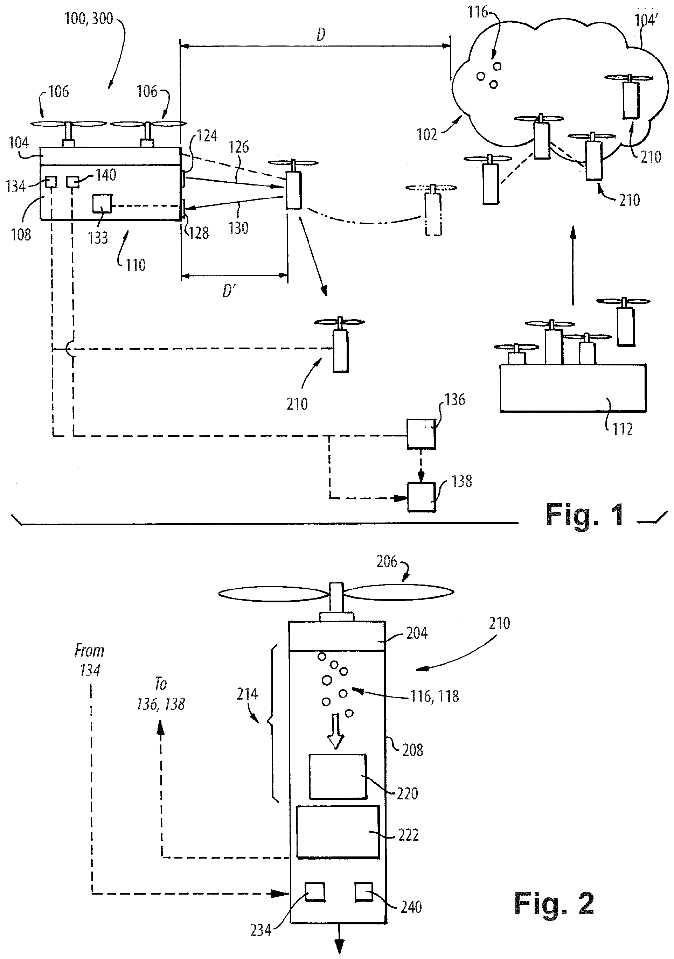

Reference will now be made to the drawings wherein like reference numerals identify similar structural features or aspects of the subject disclosure. For purposes of explanation and illustration, and not limitation, an illustrative view of an embodiment of a system in accordance with the disclosure is shown in Fig. 1 and is designated generally by reference character 100. Other embodiments and/or aspects of this disclosure are shown in Figs. 2-4. Certain embodiments described herein can be used to improve collection and detection of chemical or biological threats in the air.

In accordance with at least one aspect of this disclosure, a system 100 (e.g., for determining a chemical composition of an aerosol cloud 102) can include a moving platform 104. In certain embodiments, e.g., as shown, the moving platform can be a vehicle, such as an aircraft having one or more air movers 106 operatively connected thereto for moving the vehicle 104. In certain such embodiments, the vehicle can include any suitable aircraft, such as an airplane or a rotorcraft, and may be an unmanned aerial vehicle (UAV). Any suitable vehicle, manned or unmanned, is contemplated herein, including any suitable landcraft, aircraft, or watercraft. In certain embodiments, the moving platform can be a human or robotics. In embodiments including a vehicle, a detection module 108 can be coupled to the vehicle 104 configured to analyze a composition of an aerosol cloud 102 while the vehicle is dispatched (e.g., in flight as shown). For the ease of explanation and not limitation, the vehicle 104 and detection module 108 will be hereinafter collectively referred to as a "detection unit 110." The system 100 can also include one or more additional moving platforms 204. In certain embodiments, e.g., as shown, the moving platform can be a vehicle, such as an aircraft having one or more respective air movers 206 operatively connected thereto for moving a respective one or more vehicles 204. The one or more additional moving platforms 204 can be the same or different than that of moving platform 104. Each of the one or more additional moving platforms 204 can also include a collection module 208 coupled thereto configured to collect a sample from the aerosol cloud 102. For the ease of explanation and not limitation, the one or more additional vehicles 204 and collection modules 208 will be hereinafter collectively referred to as "collection units 210." The system 100 can also include base unit 112 configured to control dispatch of the collection units 210. In embodiments, the base unit 112 can be configured to launch a plurality, or a swarm, of collection units 210 as shown.

In embodiments, the collection module 208 can include a collection medium 214 configured to collect aerosol particles (e.g., liquid particles 116 and solid particles 118) from the aerosol cloud 102 and condense the aerosol particles on or in the collection medium 214. The collection medium 214 can be configured to collect the sample passively, for example by exposing the collection medium 214 to the aerosol cloud 102. In certain embodiments, the collection module 208 can be configured to actively collect the sample, for example, the collection module 208 can further include a pump 220 configured to draw fluid from the aerosol cloud 102 through and/or onto or into the collection medium 214. In embodiments, the collection medium 214 can include a porous surface, such as a filter medium, including filter paper for example.

In certain embodiments, the collection module 208 can also include a sensor 222 configured to detect hazardous particles in the collection medium 214 and to provide an early warning to the detection unit 110 that at least one collection unit 210 has entered a hazardous aerosol cloud. In embodiments, the sensor 222 can include any suitable sensor such as a metal oxide sensor, an optical sensor, a spectrometer, or the like. The early warning to the detection unit 110 can include a warning to maintain a predetermined standoff distance D from the aerosol cloud 102, for example as explained further below.

In embodiments, the detection module 108 of the detection unit 110 can include an optical detection module having a radiation source 124 (e.g., deep UV, infrared, quantum cascade lasers, or the like) configured to transmit an interrogation beam 126 to remotely interrogate a respective sample held within a respective collection medium 214. The optical detection module 108 can also include a radiation receiver 128 configured to receive a return signal 130 from the respective sample. The interrogation beam 142 will interrogate the sample and return one or more wavelengths (the same or different than the interrogation beam), the wavelengths being a function of the chemical composition of the sample (e.g., as in Raman spectroscopy). In embodiments, the optical detection module 108 can also include a composition determination module 132 configured to receive data from the radiation receiver 130 (e.g., an amount of light and wavelength) to determine the chemical composition of the particles in the collection medium 214, and thus the chemical composition of the aerosol cloud 102. In certain embodiments, the determination module 148 can include a spectrometer (e.g., a Raman spectrometer).

Because the composition of the aerosol cloud 102 is unknown at the time of dispatching the detection and collection units 110, 210 and before the first sample is interrogated, the standoff distance D between the detection unit 110 and the respective collection unit 210 that is being interrogated should be sufficient such that exposure of the detection unit 110 to the aerosol cloud 102 is minimized in the event the aerosol cloud contains a contaminant or hazardous material. The standoff distance may change as the aerosol cloud 102 moves or disperses and as the detection unit 110 performs different tasks. In embodiments, the standoff distance D can be determined as a function of the mobile platform 104/20 and its components. For example, if the mobile platform 104/204 includes a rotor craft (e.g., a drone), the standoff distance D can be about 10 feet, or at least far enough to avoid rotor or prop wash and drift of contamination to the optical detection module 108. For a land craft, or watercraft, the standoff distance D may be greater or less than 40 feet. Once the chemical composition of the aerosol cloud 102 has been determined (even if only preliminarily), if the aerosol cloud 102 does contain a contaminant, the standoff distance D must be maintained while sampling continues.

In embodiments, the detection unit 110 and each of the collection units 210 can each further include a respective communications module 134, 234 configured to communicate with a controller 136 to guide the units 110, 210 to one or more respective positions in space relative to the aerosol cloud 102 (e.g., geographical coordinates of interest, for example the suspected location of the aerosol cloud 102). For example, at dispatch or launch, the controller 136 can communicate with the respective communications modules 134, 234 to place the collection units 210 in or near (e.g., at an edge 102' of) the aerosol cloud 102, while the detection unit 110 can be sent to a position remote from the cloud 102, at least as far away from the cloud 102 as the standoff distance D. During the sampling process, in embodiments, the respective communications modules 134, 234 can be configured to communicate (e.g., with each other) a respective position of the detection unit 110 to the collection units 210 to maintain the standoff distance D and to maintain an appropriate interrogation distance D'. In embodiments, the respective communications modules 134, 234 can also be configured to communicate the composition of the aerosol cloud 102 to the controller 136 and/or a user 138, for example to notify on remote personnel of potential hazards with respect to the composition aerosol cloud 102. In certain embodiments, the controller 136 can be or include a remote and/or on ground controller and in certain embodiments may be included on or in the base unit 112.

In embodiments, each of the detection unit 110 and the collections units 210 can also include a respective navigation module 140, 240 configured to guide the units 110, 210 to one or more positions in space relative to the aerosol cloud 102 without communication from a remote or on ground controller (e.g. controller 136). For example, in certain embodiments, the units 110, 210 can include at least some amount autonomous control, such as with respect to positioning the units 110, 210 relative to the aerosol cloud 102 (e.g., based on sensor data from sensor 222) and/or positioning the units 110, 210 relative to other nearby units 110, 210 included in the system 100. This can include communication between units 110, 210 to position the detection unit 110 at a given location to maintain the standoff distance D as the aerosol cloud moves or disperses, communications between units 110, 210 to position the collection units 210 at an appropriate distance D' from the detection unit 110 during sampling, and/or communications between collections units 210 to avoid collisions, among others.

In embodiments, all of the units 110, 210 need to know to approach the general location of the cloud 102, at least at dispatch. However, during sampling, the detection unit 110 needs to know to stay out of and away from the cloud 102 while the collection units 210 need to know to go into the cloud 102. At the same time, the collection units 210 also need to know to come out of the cloud 102, find the detection unit 110, then standoff at the acceptable distance D' and maintain in coordination with the detection unit 110 while it analyses the sample. After sampling, the collection unit 210 also needs to know whether go back into the cloud 102 for more sampling or return to the ground or base 112 for disposal (e.g. destruction) or sample archiving.

In certain embodiments, the detection unit 110 and sampling units 210 may operate in a master/slave relationship where the detection unit 110 acts as a master and each collection unit 210 acts as a slave, such that the sampling units 210 may be controlled, at least partially, by the detection unit 110 with respect to positioning and/or flight path if not controlled autonomously or by the controller 136. In certain embodiments, the detection unit 110 and/or collection unit 210 may include some amount of autonomous or otherwise endogenous control systems for positioning purposes if communication between the detection unit 110 and the collection units 210, or between any unit 110, 210 and the controller 136, is lost, for example. Any suitable or appropriate communication system between the detection unit 110 and the collection units 210, as well as between the units 110, 210 and a grounded or otherwise remote controller 136 is contemplated herein. For example, each unit 110, 210 can have an onboard sensing or positioning system to be able to locate nearby units. It is also contemplated that the detection unit 110 can include satellite guidance that could be distributed to each of the collection unit 210.

In accordance with at least one aspect of this disclosure, a method 300 (e.g., for detecting a chemical composition of the aerosol cloud 102) can include dispatching a plurality of collection units (e.g., units 210) towards an aerosol cloud (e.g., cloud 102) and detecting, with a sensor (e.g., sensor 222) disposed within a respective collection units, an edge (e.g., edge 102') of the aerosol cloud. The method can include positioning at least one of the collection units within the aerosol cloud and collecting a sample from an aerosol cloud in a collection medium (e.g., collection medium 214) within a respective collection unit. In certain embodiments, collecting can further include passively collecting aerosol particles from the sample on or in the collection medium, or in certain embodiments, remotely collecting can include actively drawing (e.g., with a pump 220) a portion of the aerosol cloud through the collection module and onto or into the collection medium.

The method can include condensing the aerosol particles in or on the collection medium and optically interrogating, with a detection module (e.g. module 108) of a detection unit (e.g., detection unit 110), the aerosol particles in the collection medium. In embodiments, optically interrogating can include positioning the detection unit proximate the aerosol cloud but separated by a standoff distance (e.g., distance D) and positioning a respective collection unit having the collected the aerosol particles at a location between the aerosol cloud and the detection unit (e.g., at a distance D' from the detection unit). This serves to assist in preventing the detection unit from entering the aerosol cloud.

In embodiments, interrogating the samples held within the collection medium of the collection unit can occur sequentially. For example, the detection unit can call for one collection unit to approach for interrogation. After interrogation (e.g., after a few seconds), the spent collection unit can retreat and another collection unit can be called, and so forth as needed until an adequate number of samples have been interrogated.

The method can further include determining a chemical composition of the aerosol particles in the collection medium and communicating the chemical composition of the aerosol particles in the sample to a user or to a controller (e.g. to controller 136 or user 138 via communications module 134). In embodiments, e.g., after interrogation of a respective collection unit, the method can further include moving the respective collection unit away from the aerosol cloud while the optical detection system determines the chemical composition, and then re-positioning the respective collection unit in the aerosol cloud and repeating the method or homing the respective unit to a base (e.g., base 112). Once sampling is complete and all of the detection unit and collection unit are homed (e.g., return to ground if aircraft), the method can include initiating a decontamination and/or destruction protocol based at least in part of the chemical composition of the aerosol particles to decontaminate or destroy any units that are too contaminated for reuse or for archiving.

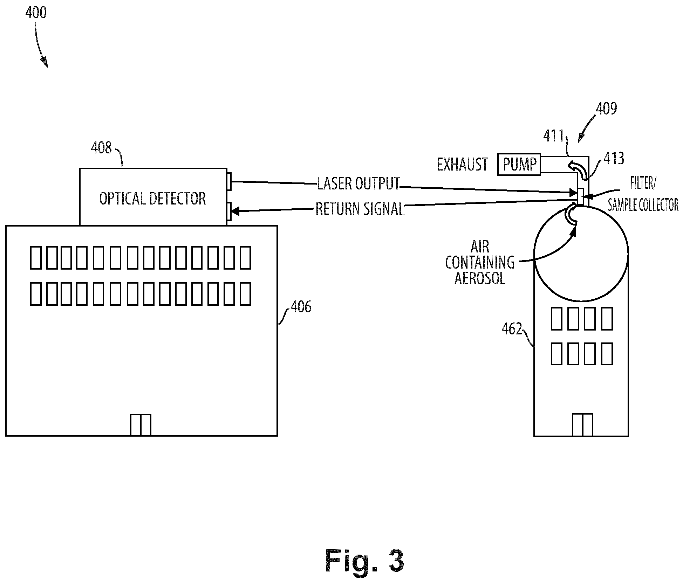

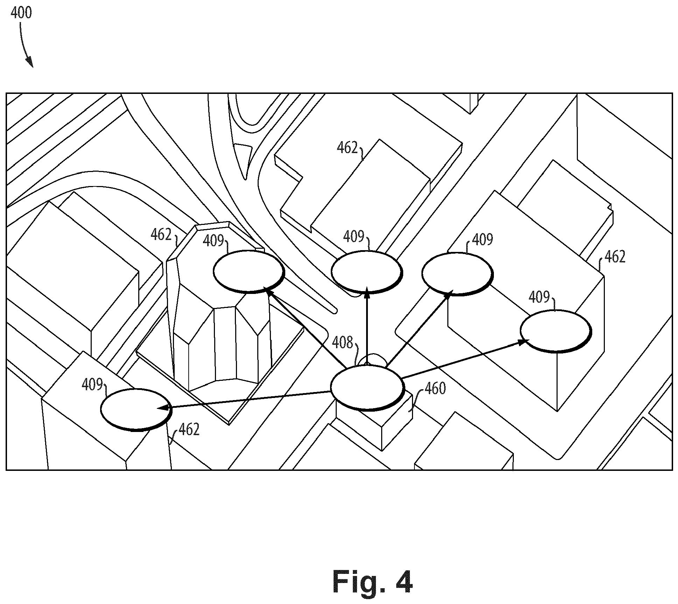

In certain embodiments, as shown in Figs. 3 and 4, a system 400 can be a stationary aerosol collection and detection system 400. The system 400 can include an optical detection module 408 and a collection unit 409, physically separate from one another. The optical detection module 308 can be the same or similar to the optical detection module 108 as described above. The collection system 409 can be the same or similar to the collection unit 210 as described above. In certain embodiments, the collection system 409 can include a collection arm 411 and collection tube 413. The system 400 can operate in the same or similar manner as to that described above with respect to system 100.

In certain embodiments, e.g., as shown, the optical detection system 408 can be included on a first stationary structure 460, and the collection system 409 can be included on a second stationary structure 462, physically separate from the first stationary structure 460 to collect aerosol samples surrounding the structures 460, 462 or from within the structures 406, 462 (e.g., in ductwork). The structure can include a building, for example if in an urban environment (e.g., as shown in Fig. 4), or any other suitable structure/fixture for a given environment. In certain embodiments, the optical detection module 408 can be included on the first stationary structure 460 while the collection system 409 can be included on a mobile platform, such as vehicle 404. In certain embodiments, the opposite can be true, where the optical detection module 408 can be included on a mobile platform such as vehicle 404, while the collection system 409 can be included the second stationary structure 462.

In embodiments, the system 400 can include any suitable number of optical detection modules 408 and any suitable number of collection system 409. In certain embodiments, the system 400 can include more collection system 409 than optical detection modules 408, and in such embodiments, the optical detection modules 408 can be centrally located (e.g., surrounded by) a plurality of collection systems 409. In certain embodiments, the first and second structures 460, 462 can include any suitable number of optical detection modules 408 or collection systems 409. For example the first structure 462 can include an optical detection module 408 and multiple collection systems 409. In certain embodiments, the second structure can include multiple collection systems 409 and no optical detection modules 408. Any suitable arrangement of optical detection modules 408 and collection systems 409 is contemplated herein.

Embodiments can utilize a hybrid approach to aerosol cloud identification by combining aspects of optically based liquid/solid chemical detection with a mobile scheme that minimizes the danger and cost of contamination of sampling/detection units. As shown and described, the systems 100, 400 and method 200 can allow for collection of aerosol particles onto a hard surface more suitable for optical detection and can keep the costly, sophisticated optical detection system out of the contaminating cloud by sacrificing smaller, cheaper units for the sample collection step.

The resulting combined mobile platform/payload for the collection units can be sufficiently cost effective to be practically disposable or at least salvageable for sample archiving and confirmatory testing.

The present disclosure contemplates the integration of the optical detection module with a moving platform in a manner that enables coordinated operation with the collection units, which may also be coupled to a moving platform. For example, unique components include those (e.g., radar, LIDAR, optical, among others) needed for the optical detection module to call or find the collection units and subsequently maintain a predefined relative position to them. In embodiments, putting the analytical payload (e.g., the detection module) in flight (or in motion generally) rather than having the sampling units return to ground for analysis allows the system 100 to minimize the time required to generate data for timely decision making by force commanders and to enable operation in contested spaces without harm to personnel.

As will be appreciated by those skilled in the art, aspects of the present disclosure may be embodied as a system, method or computer program product. Accordingly, aspects of this disclosure may take the form of an entirely hardware embodiment, an entirely software embodiment (including firmware, resident software, micro-code, etc.), or an embodiment combining software and hardware aspects, all possibilities of which can be referred to herein as a "circuit," "module," or "system." A "circuit," "module," or "system" can include one or more portions of one or more separate physical hardware and/or software components that can together perform the disclosed function of the "circuit," "module," or "system", or a "circuit," "module," or "system" can be a single self-contained unit (e.g., of hardware and/or software). Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Embodiments may include or utilize computer program instructions. These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Those having ordinary skill in the art understand that any numerical values disclosed herein can be exact values or can be values within a range. Further, any terms of approximation (e.g., "about", "approximately", "around") used in this disclosure can mean the stated value within a range. For example, in certain embodiments, the range can be within (plus or minus) 20%, or within 10%, or within 5%, or within 2%, or within any other suitable percentage or number as appreciated by those having ordinary skill in the art (e.g., for known tolerance limits or error ranges).

The articles "a", "an", and "the" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

Any suitable combination(s) of any disclosed embodiments and/or any suitable portion(s) thereof are contemplated herein as appreciated by those having ordinary skill in the art in view of this disclosure.

The embodiments of the present disclosure, as described above and shown in the drawings, provide for improvement in the art to which they pertain. While the apparatus and methods of the subject disclosure have been shown and described, those skilled in the art will readily appreciate that changes and/or modifications may be made thereto without departing from the scope of the subject disclosure.

## Claims

1. A system, comprising:
one or more collection units (210) configured to collect a sample from an aerosol cloud; and
a detection unit (110) configured to analyze a composition the sample from the aerosol cloud.

2. The system of claim 1, wherein the one or more collection units (210) includes a plurality of collection units.

3. The system of claim 1 or 2, wherein the collection unit (210) includes a collection medium (214) configured to collect aerosol particles from the aerosol cloud and condense the aerosol particles on or in the collection medium; and optionally, wherein the collection unit further includes a pump (220) configured to draw aerosol particles from the aerosol cloud onto the collection medium.

4. The system of claim 3, wherein the collection medium (214) includes a filter medium.

5. The system of claim 3 or 4, wherein the collection unit (210) further includes a sensor (222) configured to detect hazardous particles in the collection medium (214) and to provide a warning to the detection unit (110) that a respective collection unit has entered a hazardous aerosol cloud.

6. The system of claim 3, 4 or 5, wherein the detection unit (110) includes an optical detection module (108) having a radiation source configured to transmit an interrogation beam to remotely interrogate a respective collection medium of a respective collection unit, and a radiation receiver (128) configured to receive a return signal from the respective collection medium (214); and optionally, wherein the optical detection module further includes a composition determination module (132) configured to receive data from the radiation receiver to determine a chemical composition of the particles in the collection medium.

7. The system of any of claims 1 to 6, wherein a standoff distance between the detection unit (110) and the respective collection unit (210) is sufficient such that exposure of the detection unit to the aerosol cloud is minimized.

8. The system of claim 7, wherein one or more of the detection unit (110) and/or the one or more collection units (210) are configured to couple to a moving platform (204).

9. The system of claim 8, wherein the detection unit (110) and the one or more collection units (210) each further includes a respective communications module (134, 234) configured to communicate with a controller (136) to guide the detection unit and the one or more collection units to one or more respective positions in space relative to the aerosol cloud.

10. The system of claim 8, wherein the respective communications modules (134, 234) are configured to communicate a respective position of the detection unit (110) to the one or more collection units (210) to maintain the standoff distance; and/or wherein the respective communications module of the detection unit is further configured to communicate the composition of the aerosol cloud to the controller (136) and/or a user.

11. The system of any preceding claim, the detection unit (110) and the one or more collection units (210) each further include a respective navigation module (140, 240) configured to guide the detection unit and the one or more collection units to one or more positions in space relative to the aerosol cloud without communication from a remote or on-ground controller.

12. A method, comprising:
dispatching a plurality of collection units towards an aerosol cloud;
detecting, with a sensor disposed within a respective collection unit, an edge of the aerosol cloud;
collecting a sample from the aerosol cloud in a collection medium within a respective collection unit;
optically interrogating, with a detection unit, aerosol particles within the sample on or in the collection medium;
determining a chemical composition of the aerosol particles on or in the collection medium; and
communicating the chemical composition of the aerosol particles to a user.

13. The method of claim 12, wherein collecting further includes:
drawing a portion of the aerosol cloud onto a collection unit, wherein the collection unit includes the collection medium disposed therein.

14. The method of claim 12 or 13, wherein optically interrogating further includes:
positioning the detection unit having an optical detection module operatively connected thereto proximate the aerosol cloud separated by a standoff distance; and
positioning a respective collection unit having the collected aerosol particles at a location between the aerosol cloud and the detection unit.

15. The method of claim 14, further comprising:
moving the respective collection unit away from the aerosol cloud while the optical detection module determines a chemical composition of the collected aerosol particles;
re-positioning the collection unit in the aerosol cloud and repeating the method or homing the collection unit to a base; and
initiating a decontamination and/or destruction protocol based at least in part of the chemical composition of the aerosol particles.
